# EUROPEAN PATENT SPECIFICATION

(11) **EP 3 498 166 B1**
(45) Date of publication and mention of the grant of the patent: **27.01.2021**
(21) Application number: 18212289.5
(22) Date of filing: 13.12.2018
(51) Int. Cl.: A61B 5/12, A61B 5/00, H04R 25/00, A61N 1/36

(54) **DEVICE FOR DIAGNOSIS AND/OR TREATMENT OF TINNITUS AND A SYSTEM FOR ELECTRICAL AND MAGNETIC STIMULATION OF THE EAR**
VORRICHTUNG ZUR DIAGNOSE UND / ODER BEHANDLUNG VON TINNITUS UND SYSTEM ZUR ELEKTRISCHEN UND MAGNETISCHEN STIMULATION DES OHRES
DISPOSITIF DE DIAGNOSTIC ET / OU DE TRAITEMENT DE TINNITUS ET SYSTÈME DE STIMULATION ÉLECTRIQUE ET MAGNÉTIQUE DE L'OREILLE

(30) Priority: 13.12.2017 PL 42387317
(43) Date of publication of application: 19.06.2019
(73) Proprietor: Uniwersytet Medyczny w Lodzi, 90-419 Lodz (PL)
(72) Inventor: MIELCZAREK, Marzena, 92-109 Lodz (PL); OLSZEWSKI, Jurek, 91-492 Lodz (PL)
(74) Representative: Godlewski, Piotr

(56) References cited:
- CN-U- 206 660 005
- US-A1- 2003 195 588
- US-A1- 2011 295 166
- US-A1- 2015 314 124
- PHILIP KOLLMANNSBERGER ET AL: "High-force magnetic tweezers with force feedback for biological applications", REVIEW OF SCIENTIFIC INSTRUMENTS., vol. 78, no. 11, 9 November 2007 (2007-11-09), pages 1-6, XP055568090, US ISSN: 0034-6748, DOI: 10.1063/1.2804771

## Description

The object of the present invention is a magnetic field inductor, a set for diagnosing and/or treating tinnitus and a system for electric and magnetic ear stimulation.

According to the definition of the American National Standard Institute, tinnitus is a sensation of sound not being a result of external stimulation. Therefore, tinnitus is a subjective sensation - a phantom perception of sound with the lack of an acoustic stimulus in the surroundings, and it constitutes a problem in terms of both diagnostics - due to the lack of an objective method for its recording and measurement, as well as therapy - due to the lack of a therapeutic method efficient in case of every patient. In Europe approximately 70 million people claim to suffer from tinnitus. The frequency of the occurrence of tinnitus increases along with age, which can be associated with the presence and intensification of physiological changes in the auditory organ having the nature of presbycusis.

There are various scientific theories explaining the aetiology of this phenomenon. Among other things, it is assumed that tinnitus results from improper nervous activity within the auditory system, which in the auditory cortex is erroneously recognised as sound. Tinnitus originates from a place known as the tinnitus generator, where improper nervous pulsation occurs - usually this place is the cochlea. Due to degenerative changes in outer auditory cells their improper motor activity occurs, and inner auditory cells generate the impression of noise by receiving such an improper signal. The generation of noise can also be caused by impaired action of ion channels, which results in a change in the receptor potential, causing the pathological release of a neurotransmitter in the inner auditory cells or a disruption in the control of inner auditory cell movements.

Tinnitus is described by patients as ringing, squealing or buzzing, the annoyance of tinnitus being able to have a relatively wide range: from mildly annoying to highly annoying, which is usually also accompanied by headaches.

The known methods of treating tinnitus include passive methods and those which concentrate on the tinnitus generator, which include, e.g.: pharmacology, masking, electro stimulation, laser therapy, hyperbaric oxygen chambers, acupuncture and others.

Treatment by electro stimulation involves stimulation of the auditory system by a typically sinusoidal alternating current with a magnitude between 3 and 1000 µA in the form of pulses lasting for example 300 ms each at several frequencies. For each of these frequencies, the current is gradually increased up until the patient has an impression of a sound or vibration. In order to perform the procedure of electro stimulation, a working electrode is typically introduced into an ear canal filled with physiological saline.

Electro stimulation of the hearing organ probably affects the supply of blood within the inner ear or the "arrangement" of the spatial release of synaptic neurotransmitters. The stimulation current can also have an impact on facilitating the exchange of metabolites in the cell, as well as the spontaneous activity of neurons of nerve VIII. Nonetheless, the mechanism of action of electro stimulation is not entirely identified.

From the patent literature there are known various devices used in treating and diagnosing tinnitus.

From American patent application US20070213787 there is known a device for electro stimulation in tinnitus suppression therapy. The device comprises a generator of electric pulses connected to a soft spherical electrode with a diameter of between 1.5 and 2.5 mm, consisting of wire coils with a varying diameter, in the shape of a sphere. For the purpose of electro stimulation, the electrode is introduced within the round window depression in the middle ear - into the tympanic cavity, from where the electrode transmits electrical stimuli into the surroundings. Due to the soft construction of the electrode provided by the coils forming the sphere - a hollow space, the electrode causes no damage to the middle ear.

From American patent description US4622975 there is known a device for diagnosing a partial or complete loss of hearing by means of acoustic stimuli. The device has the form of an electrode for placing in the ear canal, which consists of a tube made of a conductor for receiving and conducting electric signals from the skin surface and conducting acoustic stimuli to the surface of the ear canal. The conductive tube has on its end plastic foam with conductive properties. In order to perform the examination, the conductive tube is introduced foam side first into the ear canal. The device is provided with a flange for protection against damaging the eardrum, limiting the depth of introducing the electrode inside the ear canal.

From American patent description US4706682 there is known a device for stimulating and recording electric signals. The device comprises a flexible silicone tube constituting the coating of an electrode intended to be introduced via the distal end of the ear canal. From the distal side, the tube is ended with a compressible material in the form of foam, which enables pressing it towards the eardrum with no risk of damaging it. For the purpose of electro stimulation or recording signals, the foam is saturated with conductive gel and the silicone tube is introduced into the ear canal. Subsequently, an electrode in the form of a wire is introduced into the silicone tube, so that the end of the wire touches the foam saturated with gel, providing continuity of the electrically conductive path. A reference electrode (passive) is attached in any other location on the patient's body.

Moreover, there are known methods of treating tinnitus with magnetic field, based on transcranial stimulation which uses external inductors. Typical systems for magnetic treatment of tinnitus comprise stimulators in the form of flat solenoids of relatively large sizes in order to achieve proper magnetic field strength, which are placed at a small distance from the head. However, electromagnetic inductors of this type do not allow the concentration of magnetic field near the cochlea, which contributes to unsatisfactory results of tinnitus treatment produced by means of this method.

A publication "High-force magnetic tweezers with force feedback for biological applications" (by Philip Kollmannsberger et al, Review of Scientific Instruments, vol. 78, no. 11, 9 November 2007, pages 1-6) discloses magnetic tweezers suitable for magnetic micromanipulation or microrheology techniques that comprise a magnetic microneedle that consists of a cylindrical rod made of high-permeability nickel alloy.

From American patent application US2003195588 there is known a system for treating various neurological, vestibular, and other disorders that includes a stimulator device situated in an ear canal of the patient. The stimulator device is adapted to provide magnetic, electrical, audible, tactile, or caloric stimulation, and may be programmed to provide such stimulation in continuous, semi-continuous, periodic, programmed, or on-demand modes, or various combinations of the above.

It would be purposeful to develop a device enabling the diagnosis and treatment of tinnitus by means of magnetic field, with a construction providing increased concentration of magnetic field near the cochlea, by doing so providing an alternative or complement other tinnitus treatment techniques, such as for example electro stimulation.

The object of the invention is a magnetic field inductor for diagnosing and/or treating tinnitus, a set for diagnosing and/or treating tinnitus and a system for electric and magnetic ear stimulation according to the appended claims.

The object of the invention is presented in an embodiment in the drawing, in which:
Fig. 1 presents schematically a magnetic field inductor in a lengthwise cross-section through the ferromagnetic core;
Fig. 2 presents schematically the magnetic field inductor placed in the ear canal;
Fig. 3 presents schematically the magnetic field inductor placed in the ear canal - magnified;
Fig. 4A presents schematically a set for diagnosing and treating tinnitus comprising a magnetic field inductor;
Fig. 4B presents schematically the inductor in a plastic coating;
Fig. 5 presents the results of simulation of magnetic field induction distribution near the inductor, against the anatomical structures of the ear, for an effective frequency of 20 Hz and an effective inductor current value of 3.5 A;
Fig. 6 presents the flow chart of a system for electric and magnetic ear stimulation.

The magnetic field inductor according to the invention is intended in particular to treat tinnitus using variable magnetic field. The developed construction of the inductor enables its placement directly in the ear - in the ear canal, as well as simultaneous stimulation of the vicinity of the cochlea and the cochlear nerve with magnetic field.

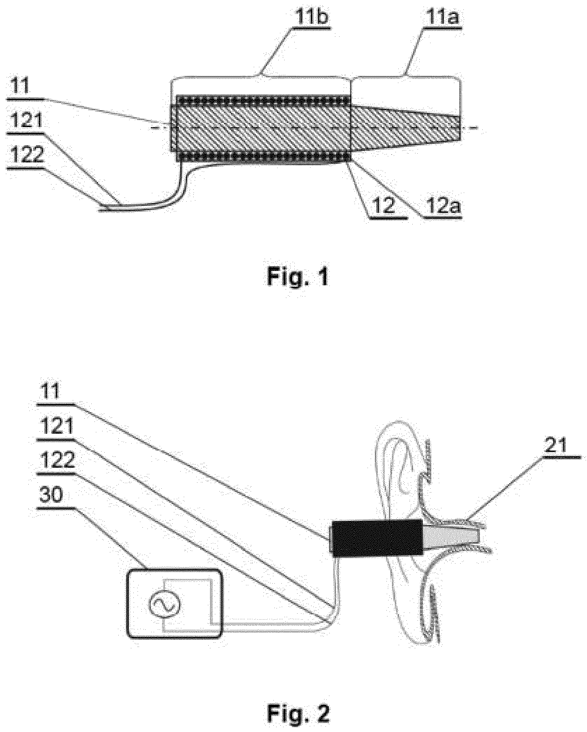

Fig. 1 presents the magnetic field inductor in a lengthwise cross-section. The inductor comprises a longitudinal ferromagnetic core 11. Preferably, the core 11 is made of a soft ferromagnet (i.e. a ferromagnet which loses its outer magnetisation upon removing the magnetic field, retaining only residual magnetisation considerably lower than the maximum). For example, a ferromagnet with magnetic permeability of the material of µ=1800 can be used. For example, the 3F3 material from the Ferroxcube/Yageo company (USA) may be used, ensuring small losses and high field strength value, at which there is saturation amounting to approximately 50 A/m.

The core 11 comprises a proximal section 11 b, preferably cylindrical, and a distal section 11a (preferably conical), which sections: 11a, 11b are connected coaxially - one behind the other, the distal section 11a having a diameter decreasing in a direction away from the proximal section 11b.

The conical distal section 11a of the core has a base connected to the proximal section 11b, and a free end with a smaller diameter than the base, which can have truncated or rounded edges, in order to facilitate the placement of the core in the patient's ear.

Over at least a partial length of the cylindrical proximal section 11b, the core 11 has a conductor solenoid 12 wound onto it forming coils 12a. For example, the solenoid 12 can have 230 coils of a wire with a diameter of 0.3 mm. The ends 121, 122 of the solenoid are adapted to be connected to an electric generator 30.

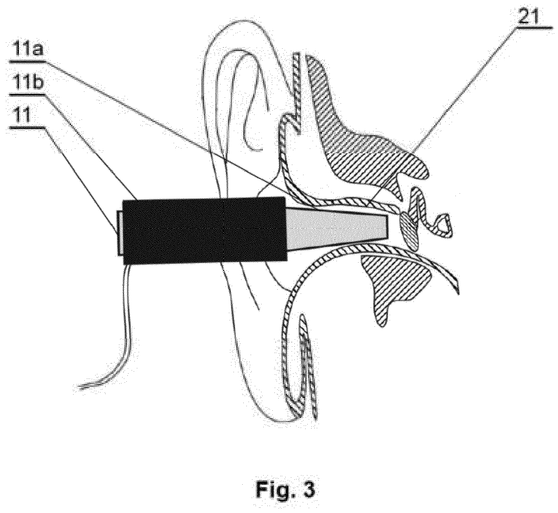

Fig. 2 and Fig. 3 in magnification present schematically a method for placing the magnetic field inductor in the patient's ear. The construction of the core is designed such that the ferromagnetic core 11 is partially introduced into the patient's ear canal, the distal section 11a of the ferromagnetic core being intended for introduction into the ear canal 21, while the proximal section 11a with the solenoid 12 wound onto it is intended for protruding outside the patient's body - beyond the ear canal 21. The conical construction of the distal section 11a provides an increased contact surface of the conical section of the core 11 with the surface of the ear canal; in other words: the distal section 11a of the ferromagnetic core 11 fills at least partially the ear canal, while touching the walls of this canal. Such a construction of the distal section 11a enables the performance of both the diagnosis and treatment without the necessity to fill the patient's ear canal with physiological saline, and therefore without the necessity to perform stimulation in the patient's lying position. Due to the conical construction of the distal section 11a of the core 11 for introduction into the ear canal, the surface of the core 11 within the distal section 11a abuts directly the cells forming the wall of the ear canal. Due to this, during stimulation the patient can remain in a convenient standing, sitting or lying position.

Due to the fact that upon placing the ferromagnetic core 11 in the ear canal the conductive solenoid 12 is placed outside the ear canal, increased induction of the magnetic field near the middle ear is possible.

The magnetic field inductor provides the achievement of a technical effect in the form of increased concentration of magnetic field near the stimulated structures, that is the cochlea and the patient's cochlear nerve - compared to conventional techniques of transcranial therapy with magnetic field, in which the conductive solenoid is positioned at a certain distance from the head, usually at the top of the head. This effect has been achieved by the developed construction of the device, in which the conductive solenoid is wound solely onto the proximal section of the ferromagnetic core meant to be placed outside the ear - beyond the patient's ear canal, while the distal section 11a of the core to be introduced into the ear canal does not comprise a wound conductive solenoid and it has a conical shape.

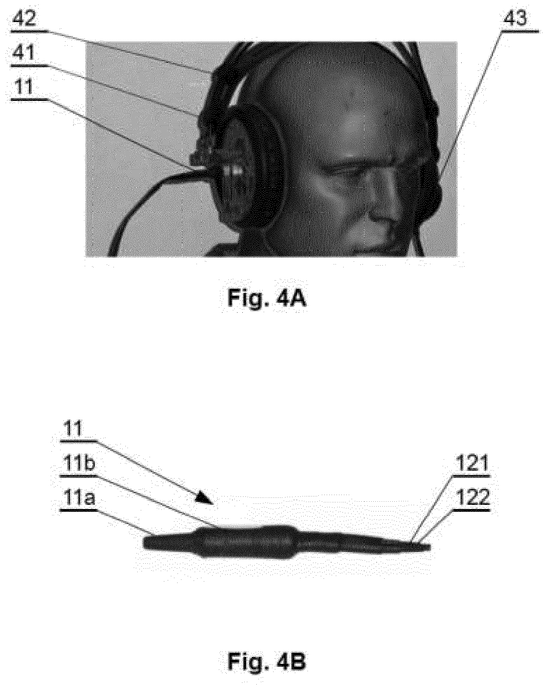

Fig. 4A presents by way of example a set for diagnosing and treating tinnitus. The set comprises a magnetic field inductor 11 attached within a handle 41, an audio earpiece 43 for generating acoustic signals, and a headband 42 for connecting the handle of the magnetic field inductor 41 to the audio earpiece 43, in order to properly attach the set onto the patient's head - with an inductor placed in the patient's ear in a manner ensuring the introduction of the distal section 11a of the ferromagnetic core 11 into the ear canal and the proximal section 11b of the ferromagnetic core outside - beyond the ear canal, and the audio earpiece 43 placed at the opposite ear of the patient. The handle 41 for stabilising the magnetic field inductor 11 can have various designs, for example as presented in Fig. 4A - the handle 41 can have the construction of a flange limiting the depth of introduction of the inductor inside the ear canal. Such a construction of the set eliminates the necessity for the patient to hold the inductor inside the ear by themselves.

As presented in Fig. 4B, the inductor can have a coating, preferably made of plastic, covering the surface of the core 11 comprising a proximal section 11b with a conductive solenoid and a distal section 11a, as well as the ends of the solenoid 121, 122 adapted to be connected to an electric generator 30.

When diagnosing and/or treating tinnitus, the patient puts the set by themselves or with the help of a specialist on their head, so that the audio earpiece 43 is positioned at one ear, while the inductor is properly placed in the ear canal of the opposite ear. During stimulation, the audio earpiece transmits to the patient's ear sounds which the patient by themselves matches with their own tinnitus. This technique will potentially result in the most precise mapping of the psycho acoustic parameters of tinnitus, which will be subsequently used in selecting the parameters of magnetic / electric ear stimulation for their treatment. One of the possibilities of selecting the band of the stimulating factor involves its adjustment to the range of damaged frequencies of the hearing organ. Due to the fact that for most patients tinnitus is considered as a sign of damage of specific frequencies of hearing (it lies in the band of damaged frequencies of hearing), the stimulation of these very frequencies is found justified in treatment. Therefore, the precise determination of the tinnitus band will allow individual selection of the frequency of the stimulating factor.

During diagnosis based on the type and intensity of sounds selected by the patient, the type and level of annoyance of noises are determined. The magnetic method of diagnosis can also facilitate or enable discerning the origin of tinnitus as peripheral and central.

For example, the magnetic field generated by the solenoid can have the following parameters: frequency 20-20000Hz, magnetic induction - 36-28000 microT.

### AN EMBODIMENT

The core has been made from the 3F3 material as a lengthwise element with a circular cross-section. Core dimensions: total length: 49 mm, length of the proximal section: 30 mm, diameter of the proximal section over the whole length of said section: 7.9 mm, length of the conical distal section: 19 mm, the largest diameter of the conical distal section: 7.8 mm, the smallest diameter of the conical distal section: 4 mm, diameter of the proximal section with the solenoid: 10 mm. The solenoid has been made by winding 230 coils of a wire with a diameter of 0.3 mm onto the core.

An inductor with the abovementioned dimensions has been placed in the ear canal and stabilised on the patient's head by means of a handle in the form of a flange connected to the headband.

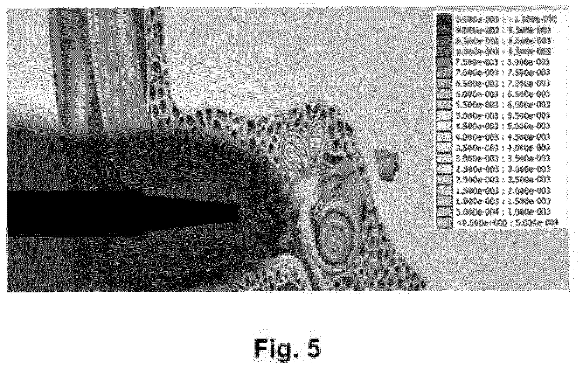

Results of the simulation of magnetic induction produced assuming the frequency of current flowing in the solenoid f = 20Hz and a current of i = 3.5 are presented in Fig. 5. Based on the performed simulation it can be concluded that magnetic induction near the cochlea, that is at a distance of 10 mm from the end of the core placed in the ear canal, amounted to B = 6 mT.

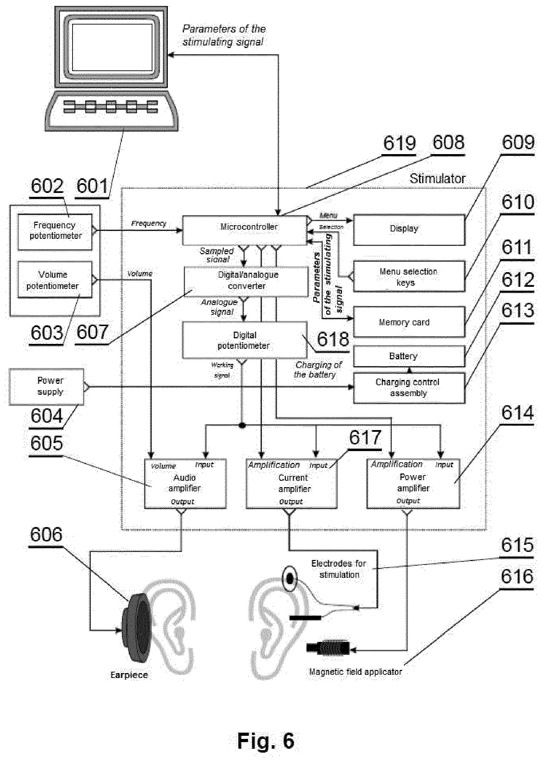

Fig. 6 presents a flow chart of a system for electric and magnetic ear stimulation. The system is meant for electro stimulation and magnetostimulation of the hearing organ for patients with tinnitus, as well as extending the diagnostics of the hearing system and tinnitus in this group of patients.

The system according to the invention enables extended control of the parameters of signals used for stimulation and it allows individual selection of the conditions of stimulation.

The system is intended for the performance of electro stimulation by means of a non-invasive method - hydro transmission. In this method a stimulating electrode 615 is placed in the ear canal, filled with a physiological saline solution. The passive electrode is placed on the forehead in the anterior median line (this can be a typical electrode used for EKG, attached to degreased forehead skin).

Stimulation by means of magnetic field is performed by means of an inductor 616, according to the present invention, placed in the ear canal, due to which the highest magnetic field strength is present near the eardrum and the cochlea.

A system for electric and magnetic ear stimulation for clinical use consists of the following elements: a hardware or software controller 601; a stimulator 619; an earpiece provided with an electrode for electro stimulation 615; a magnetic field inductor 616 according to present invention.

The controller 601 enables defining the parameters of signals used for electric stimulation or magnetic stimulation. Signals defined in the software are transmitted to the stimulator 619 which generates proper current flows. In the case of conducting electrical stimulation, signals generated by the stimulator are connected to the stimulating (ear) and passive (forehead) electrodes 615. In the case of magnetic stimulation, signals generated by the stimulator are connected to a magnetic field inductor 616 placed in the ear canal.

Communication between the controller 601 and the stimulator 619 can proceed by wire or wirelessly (for example, by using interfaces such as Bluetooth or WiFi).

The stimulator 619 is preferably a device powered by a battery 612, which generates electric signals defined by the controller 601. Besides the battery 612, the stimulator is preferably provided with a charging control assembly 613 and suitable connection to the mains supply 604. In an alternative embodiment, the stimulator 619 can be powered from a mains supply and does not have a battery 612 and a charging control assembly 613.

The signals can be used for electro stimulation, magnetostimulation and acoustic stimulation. It is possible to change the amplitude of generated signals, and in the case of a sinusoidal signal, also its frequency.

The earpiece 606 enables the generation of sounds constituting reference auditory stimuli. Due to its location on the head, it is preferable to use the earpiece 606 also as a handle for attaching the active electrode and the inductor 616 in the ear canal. For this reason, it can be used even in a case in which reference sounds will not be used. The wires for connecting the electrodes can be integrated with the earpiece, and the wires for connecting the inductor can be independent of the earpiece.

The operating parameters of the electrode for electro stimulation 615 can be controlled by means of the user's manipulator having a frequency potentiometer 602 which enables changing the frequency of the signal, and by means of a volume potentiometer 603 which enables changing the amplitude of the signal.

The signal from the volume potentiometer 603 is delivered to the volume input of the audio amplifier 605 (generating audio signal for the earpiece 606), which receives audio data from the digital potentiometer 618 controlled by an analogue-to-digital converter 607, which in turn is controlled by the micro-controller 608 of the stimulator 619.

The signal from the frequency potentiometer 602 is transmitted to the microcontroller 608. The micro-controller 608 of the stimulator 619 receives control signals from the main controller 601. The micro-controller 608 of the stimulator 619 can also cooperate with the display 609 meant for communication with the user.

The display 609 can be a touch screen, in order to enable the user to enter instructions easily. If the display 609 is not a touch screen, then the stimulator 619 can be provided with buttons 610 on its housing meant for selecting desired options for the stimulator 619.

Moreover, the micro-controller 608 of the stimulator 619 is responsible for controlling the amplification of the current amplifier 617 for stimulating the electrode 615. The current amplifier 617 is also controlled by the output of the digital potentiometer 618.

The micro-controller 608 of the stimulator 619 also controls the amplification of the power amplifier 614 responsible for stimulating the magnetic field applicator 616. The power amplifier 614 is also controlled by the output of the digital potentiometer 618.

As shown in Fig. 6, amplifiers 605, 617, 614 can be controlled by the same operating signal of the digital potentiometer 618.

Moreover, the micro-controller 608 of the stimulator 619 can be adjusted to downloading the parameters of the stimulating signal from memory 611, which can have the form of a memory card or any other non-volatile memory.

## Claims

1. A magnetic field inductor for diagnosing and/or treating tinnitus, comprising a ferromagnetic core with a conductor solenoid wound onto it, **characterised in that** the elongated ferromagnetic core (11) comprises a proximal section (11b) for placing outside the ear canal (21) and connected to a distal section (11a) for introduction into the ear canal (21) and having a shape of a truncated cone, wherein a diameter of the distal section (11a) decreases in a direction away from the proximal section (11b), and wherein the conductor solenoid (12) is wound onto the proximal section (11a) along at least a part of the length of the proximal section (11a).

2. The inductor according to any of the preceding claims **characterised in that** the ferromagnetic core (11) has a circular transverse cross-section over its whole length.

3. The inductor according to any of the preceding claims **characterised in that** the proximal section (11b) of the ferromagnetic core (11) has a cylindrical shape.

4. The inductor according to any of the preceding claims **characterised in that** the ferromagnetic core is made of a material with magnetic permeability of *µ*=1800.

5. The inductor according to any of the preceding claims **characterised in that** the ferromagnetic core (11) is made of a soft ferromagnet.

6. The inductor according to any of the preceding claims **characterised in that** the solenoid (12) has 230 coils of a wire with a diameter of 0.3 mm.

7. A set for diagnosing and/or treating tinnitus comprising a magnetic field inductor (616) according to any of the claims 1 to 6.

8. The set according to claim 7 **characterised in that** it comprises a handle (41) for attaching the inductor.

9. The set according to claim 8 **characterised in that** the handle (41) has the construction of a flange for limiting the depth of introduction of the inductor inside the ear canal (21).

10. The set according to any one of the claims from 7 to 9 **characterised in that** it comprises a headband (42) for stabilising the inductor in the ear canal.

11. The set according to any one of the claims from 7 to 10 **characterised in that** it comprises an audio earpiece (43) for generating acoustic signals.

12. The set according to claim 11 **characterised in that** the audio earpiece (43) and the inductor are attached to the headband (42) at its opposite ends.

13. The set according to claim 11 **characterised in that** the audio earpiece and the flange (41) are attached to the headband (42) at its opposite ends.

14. A system for electric and magnetic ear stimulation comprising a magnetic field inductor (616); an electrode for electro stimulation (615); a stimulation controller (601); and a stimulator (619) stimulating the magnetic field inductor (616) and the electrode for electro stimulation (615) according to information received from the stimulation controller (601); **characterised in that** it comprises a magnetic field inductor (616) according to any of the claims from 1 to 6.

## Patentansprüche

1. Ein Magnetfeldinduktor zur Diagnose und/oder Behandlung von Tinnitus, umfassend einen ferromagnetischen Kern mit einer darauf gewickelten Leiterspule, **dadurch gekennzeichnet, dass** der längliche ferromagnetische Kern (11) einen proximalen Abschnitt (11b) zur Anordnung außerhalb des Gehörgangs (21) umfasst, der mit einem distalen Abschnitt (11a) zur Einführung in den Gehörgang (21) verbunden ist und die Form eines Kegels aufweist, wobei ein Durchmesser des distalen Abschnitts (11a) in die Richtung des proximalen Abschnitts (11b) abnimmt und wobei die Leiterspule (12) auf dem proximalen Abschnitt (11a) entlang mindestens eines Teils der Länge des proximalen Abschnitts (11a) aufgewickelt ist.

2. Ein Induktor nach einem der vorstehenden Ansprüche, **dadurch gekennzeichnet, dass** der ferromagnetische Kern (11) über seine gesamte Länge einen kreisförmigen Querschnitt aufweist.

3. Ein Induktor nach einem der vorstehenden Ansprüche, **dadurch gekennzeichnet, dass** der proximale Abschnitt (11b) des ferromagnetischen Kerns (11) eine zylindrische Form aufweist.

4. Ein Induktor nach einem der vorstehenden Ansprüche, **dadurch gekennzeichnet, dass** der ferromagnetische Kern aus einem Material mit einer magnetischen Permeabilität von µ=1800 ausgeführt ist.

5. Ein Induktor nach einem der vorstehenden Ansprüche, **dadurch gekennzeichnet, dass** der ferromagnetische Kern (11) aus einem weichen Ferromagneten ausgeführt ist.

6. Ein Induktor nach einem der vorstehenden Ansprüche, **dadurch gekennzeichnet, dass** die Spule (12) 230 Windungen aus einem Draht mit einem Durchmesser von 0,3 mm aufweist.

7. Eine Vorrichtung zur Diagnose und/oder Behandlung von Tinnitus, umfassend einen Magnetfeldinduktor (616) nach einem der Ansprüche 1 bis 6.

8. Eine Vorrichtung nach Anspruch 7, **dadurch gekennzeichnet, dass** sie einen Griff (41) zur Befestigung des Induktors umfasst.

9. Eine Vorrichtung nach Anspruch 8, **dadurch gekennzeichnet, dass** der Griff (41) als Flansch zur Begrenzung der Einführtiefe des Induktors in den Gehörgang (21) ausgebildet ist.

10. Eine Vorrichtung nach einem der Ansprüche von 7 bis 9, **dadurch gekennzeichnet, dass** sie ein Kopfband (42) zur Stabilisierung des Induktors im Gehörgang umfasst.

11. Eine Vorrichtung nach einem der Ansprüche 7 bis 10, **dadurch gekennzeichnet, dass** sie ein Audiogerät (43) zur Erzeugung akustischer Signale umfasst.

12. Eine Vorrichtung nach Anspruch 11, **dadurch gekennzeichnet, dass** das Audiogerät (43) und der Induktor an seinen gegenüberliegenden Enden am Kopfband (42) befestigt sind.

13. Eine Vorrichtung nach Anspruch 11, **dadurch gekennzeichnet, dass** das Audiogerät und der Flansch (41) an seinen gegenüberliegenden Enden am Kopfband (42) befestigt sind.

14. Ein System zur elektrischen und magnetischen Stimulation des Ohres mit einem Magnetfeldinduktor (616); einer Elektrode zur Elektrostimulation (615); einer Stimulationssteuerung (601) und einem Stimulator (619), der den Magnetfeldinduktor (616) und die Elektrode zur Elektrostimulation (615) gemäß der von der Stimulationssteuerung (601) erhaltenen Information stimuliert; **dadurch gekennzeichnet, dass** es einen Magnetfeldinduktor (616) gemäß einem der Ansprüche 1 bis 6 enthält.

## Revendications

1. Inducteur de champ magnétique pour diagnostiquer et/ou traiter les acouphènes, comprenant un noyau ferromagnétique avec un solénoïde conducteur enroulé dessus, **caractérisé en ce que** le noyau ferromagnétique allongé (11) comprend une section proximale (11b) destinée à être placée à l'extérieur du conduit auditif (21) et reliée à une section distale (11a) pour introduction dans le conduit auditif (21) et ayant une forme de tronc de cône, dans lequel un diamètre de la section distale (11a) diminue dans une direction s'éloignant de la section proximale (11b), et dans lequel le solénoïde conducteur (12) est enroulé sur la section proximale (11a) le long d'au moins une partie de la longueur de la section proximale (11a).

2. Inducteur selon l'une quelconque des revendications précédentes, **caractérisé en ce que** le noyau ferromagnétique (11) a une section transversale circulaire sur toute sa longueur.

3. Inducteur selon l'une quelconque des revendications précédentes, **caractérisé en ce que** la section proximale (11b) du noyau ferromagnétique (11) a une forme cylindrique.

4. Inducteur selon l'une quelconque des revendications précédentes, **caractérisé en ce que** le noyau ferromagnétique est fait d'un matériau dont la perméabilité magnétique est de µ = 1800.

5. Inducteur selon l'une quelconque des revendications précédentes, **caractérisé en ce que** le noyau ferromagnétique (11) est fait d'un matériau ferromagnétique doux.

6. Inducteur selon l'une quelconque des revendications précédentes, **caractérisé en ce que** le solénoïde (12) comporte 230 bobines d'un fil d'un diamètre de 0,3 mm.

7. Ensemble pour diagnostiquer et/ou traiter les acouphènes comprenant un inducteur de champ magnétique (616) selon l'une quelconque des revendications 1 à 6.

8. Ensemble selon la revendication 7, **caractérisé en ce que** il comprend une anse (41) pour la fixation de l'inducteur.

9. Ensemble selon la revendication 8, **caractérisé en ce que** l'anse (41) a la construction d'une collerette pour limiter la profondeur d'introduction de l'inducteur à l'intérieur du conduit auditif (21).

10. Ensemble selon l'une quelconque des revendications 7 à 9, caractérisé en qu' il comprend un bandeau (42) pour stabiliser l'inducteur dans le conduit auditif.

11. Ensemble selon l'une quelconque des revendications 7 à 10, caractérisé en qu' il comprend un écouteur audio (43) pour générer des signaux acoustiques.

12. Ensemble selon la revendication 11, **caractérisé en ce que** l'écouteur audio (43) et l'inducteur sont fixés au bandeau (42) à ses extrémités opposées.

13. Ensemble selon la revendication 11, **caractérisé en ce que** l'écouteur audio et la collerette (41) sont fixés au bandeau (42) à ses extrémités opposées.

14. Système de stimulation auditive électrique et magnétique comprenant un inducteur de champ magnétique (616) ; une électrode pour l'électrostimulation (615) ; un contrôleur de stimulation (601) ; et un stimulateur (619) stimulant l'inducteur de champ magnétique (616) et l'électrode pour l'électrostimulation (615) en fonction des informations reçues du contrôleur de stimulation. (601) ; **caractérisé en ce que** il comprend un inducteur de champ magnétique (616) selon l'une quelconque des revendications 1 à 6.
